(19) Europäisches Patentamt
European Patent Office
Office européen des brevets

(11) EP 0 556 737 B1

(12) # EUROPÄISCHE PATENTSCHRIFT

(45) Veröffentlichungstag und Bekanntmachung des Hinweises auf die Patenterteilung:
**12.01.2000 Patentblatt 2000/02**

(51) Int. Cl.$^7$: **C07D 213/61**

(21) Anmeldenummer: **93102219.8**

(22) Anmeldetag: **12.02.1993**

(54) **Verfahren zur Herstellung von substituierten 2,3-Difluorpyridinen**

Process for the preparation of substituted 2,3-difluoro-pyridines

Procédé pour la préparation de 2,3-difluoropyridines substituées

(84) Benannte Vertragsstaaten:
**CH DE FR GB LI**

(30) Priorität: **20.02.1992 DE 4205170**

(43) Veröffentlichungstag der Anmeldung:
**25.08.1993 Patentblatt 1993/34**

(73) Patentinhaber: **Clariant GmbH**
**65929 Frankfurt am Main (DE)**

(72) Erfinder:
- **Pfirmann, Ralf, Dr.**
  **D-6103 Griesheim (DE)**
- **Papenfuhs, Theodor, Dr.**
  **D-6000 Frankfurt am Main 50 (DE)**

(56) Entgegenhaltungen:
**EP-A- 0 104 715** **EP-A- 0 120 575**
**EP-A- 0 146 924** **EP-A- 0 192 287**

- **CHEMICAL ABSTRACTS, vol. 117, no. 23, 7. Dezember 1992, Columbus, Ohio, US; abstract no. 233865k, S. KUMAI ET AL 'Preparation of fluorinated pyridines.' Seite 858 & JP-A-04 164 068 (ASAHI GLASS., LTD.)**

Bemerkungen:
Die Akte enthält technische Angaben, die nach dem Eingang der Anmeldung eingereicht wurden und die nicht in dieser Patentschrift enthalten sind.

EP 0 556 737 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein neues Verfahren zur Herstellung von substituierten 2,3-Difluorpyridinen, die vor allem Bedeutung als Vorprodukte für Agrochemikalien und Pharmazeutika besitzen.

Im Gegensatz zu durch weitere aktivierende Gruppen, wie mit Trifluormethyl-, Nitro- oder Cyanogruppen substituierten Pyridinen (vgl. EP-Offenlegungsschrift 146 924) gelingt beispielsweise beim 2,3,5-Trichlorpyridin der Austausch des Chloratoms in der 3-Position nur recht schwer, da der Nachbargruppeneffekt, der durch das zuerst in die 2-Position eingetretene Fluoratom hervorgerufen wird, ziemlich gering ist.

Der Austausch der beiden Chloratome zum 5-Chlor-2,3-difluorpyridin, ebenso wie auch bei anderen substituierten 2,3-Dichlorpyridinen, die sich je nach Reaktivität aufgrund des jeweiligen Substitutionsmusters stark unterscheiden können, erfolgt jedoch in befriedigenden Ausbeuten, wenn man das Produkt direkt nach dessen Entstehen abdestilliert (halbkontinuierliche Verfahrensweise). In der genannten EP-Offenlegungsschrift 146 924 wird aus Reaktivitätsgründen im technisch unerwünschten, für die Halex-Reaktion aber vorteilhaften Lösungsmittel Dimethylsulfoxid unter gleichzeitigem Einsatz von vorzugsweise reinem Cäsiumfluorid unter ständigem Abdestillieren des Produktes gearbeitet. Trotz dieser für diesen Reaktionstyp sehr günstigen, aber unwirtschaftlichen und ökologisch bedenklichen Reaktionsbedingungen wird dennoch nur eine Ausbeute von weniger als 55 % d.Th. erhalten (siehe das nachfolgende Vergleichsbeispiel 1; siehe auch die EP-OS 146 924, Beispiel 11) . Technisch vorteilhafter wegen der geringeren Apparatekosten wäre jedoch ein vollständig batchweise durchführbares Verfahren ohne ständiges Abdestillieren des Produktes. Wegen der geringen Beständigkeit des Produktes unter den Reaktionsbedingungen und des niedrigen Siedepunktes desselben, was zu Dichtigkeitsproblemen in den verwendeten Apparaturen führen kann, schien bis jetzt ständiges Abdestillieren des gerade entstandenen Produktes sinnvoll und notwendig, wie dies in der EP-OS 146 924 beschrieben ist.

Setzt man Kaliumfluorid, auch in großem Überschuß, in Sulfolan als Lösungsmittel ein, so beträgt die Ausbeute (GC) an 5-Chlor-2,3-difluorpyridin nach 24 Stunden nur 5 % d. Th.; der Umsatz etwa 50 %. Die Bildungsreaktion von 5-Chlor-2,3-difluorpyridin schreitet also recht langsam voran. Wegen der geringen Bildungsgeschwindigkeit gelingt kontinuierliches Abdestillieren nur schwierig. Einfacher ist dies noch bei reaktiveren Substraten, wie zum Beispiel 3-Chlor-2-fluor-5-trifluormethylpyridin (siehe EP-OS 146 924, Beispiel 7), wobei aber auch hier eine apparative Vereinfachung und eine erhöhte Raumleistung ohne Ausbeuteverschlechterung wünschenswert wären.

Wenn man bei diesem reaktiven Substrat 3-Chlor-2-fluor-5-trifluormethylpyridin den Chloraustausch gemäß der EP-OS 104 715 ohne ständiges Abdestillieren durchführt, so erfordert dies den Einsatz des teuren Cäsiumfluorids. Selbst mit diesem reaktiven Substrat und reaktiven Fluorid ist die Ausbeute noch nicht befriedigend (vgl. diese EP-OS 104 715, Beispiel 2: 48 % d. Th.).

Bekannt ist aus der DE-Offenlegungsschrift 3 700 764 weiterhin die Herstellung von 2,3-Difluor-5-chlorpyridin über 2,5-Dichlorpyridin durch Nitrierung dieses Produktes sowie Reduktion zum 2,5-Dichlor-3-aminopyridin, anschließende Nitrierung zum Nitraminoderivat und Behandein desselben mittels Bortrifluorid-etherat, wobei 2,5-Dichlor-3-fluorpyridin erhalten wird. Dieses kann dann durch Austauschreaktion mit Kaliumfluorid und/oder Cäsiumfluorid in das gewünschte Produkt überführt werden. Dieser recht lange Weg weist als Nachteile ungenügende Ausbeuten, hohe Toxizität und Explosivität der Zwischenprodukte sowie die Verwendung des technisch unerwünschten Reagenzes Bortrifluorid auf.

Die Chlor/Fluor-Austausch-Reaktion bei gleichzeitiger denitrierender Fluorierung von 2,5-Dichlor-3-nitropyridin, die ebenfalls literaturbekannt ist (DE-Offenlegungsschrift 3 700 779) liefert ebenfalls ungenügende Ausbeuten. Zudem ist der Austausch von Nitrogruppen mit Problemen bei der Reaktionsführung (nitrose Gase) sowie mit erheblichen Anteilen an Nebenreaktionen verbunden, die nur schwer unterdrückt werden können. Daher war bisher der Einsatz von teurem Cäsiumfluorid empfehlenswert.

Nach der EP-Offenlegungsschrift 178 260 kann 2,3-Difluor-5-chlorpyridin ausgehend von 2,5-Dichlor-3-nitropyridin durch Hydrierung der Nitrogruppe, anschließende Diazotierung und Austausch der Diazogruppe in Fluorwasserstoff in einem Monel-Autoklaven und nachfolgender Halex-Reaktion zur Einführung des Fluoratoms in der 2-Position mit Kaliumfluorid und Cäsiumfluorid (6:1) hergestellt werden. Bei diesem Prozeß werden bessere Ausbeuten erhalten, jedoch ist auch dieses Verfahren vielstufig und bringt Korrosionsprobleme bei der Handhabung von wasserfreiem Fluorwasserstoff mit sich (unwirtschaftliche Apparaturen); außerdem kann es wirtschaftlich nicht mit der Einführung beider Fluoratome in einem Schritt durch preisgünstiges Alkalimetallfluorid konkurieren.

Aufgabe der vorliegenden Erfindung war es daher, die Nachteile des Standes der Technik zu vermeiden und insbesondere ein Verfahren zur Herstellung von substituierten 2,3-Difluorpyridinen bereitzustellen, das apparativ einfach durchzuführen ist und ohne ständiges Abdestillieren des hergestellten substituierten 2,3-Difluorpyridins auskommt, dennoch zufriedenstellende Ausbeuten und hohe Raumleistungen bei somit niedrigen Apparatekosten liefert und das den Einsatz von großen Mengen an reinem Cäsiumfluorid vermeidet.

gelöst wird diese Aufgabe durch ein Verfahren zur Herstellung von substituierten 2,3-Difluorpyridinen in der Schmelze oder in Anwesenheit eines polar aprotischen Lösungsmittels oder eines unpolaren Lösungsmittels, mit Ausnahme von Benzonitril, das dadurch gekennzeichnet ist, daß man substituierte 2,3-Dihalogenpyridine aus der Gruppe substituierte 2,3-Dichlorpyridine, substituierte 2-Chlor-3-fluorpyridine oder substituierte 3-Chlor-2-fluorpyridine mit Kalium-, Rubidium- oder Tetraalkyl ($C_1$-$C_{18}$)ammoniumfluorid oder deren Mischungen in Mischung mit Cäsiumfluorid, wobei der Anteil an Cäsiumfluorid nicht mehr als 50 Gew.-% beträgt, in Anwesenheit von Phasentransferkatalysatoren bei Temperaturen zwischen 120°C und 250°C umsetzt, ohne die erhaltenen substituierten 2,3-Difluorpyridine ständig aus der Reaktionsmischung zu entfernen.

Die im erfindungsgemäßen Verfahren eingesetzten substituierten 2,3-Halogenpyridine sind in der Regel ein- oder zweifach, vorzugsweise einfach substituiert. Vorzugsweise besitzen diese substituierten 2,3-Halogenpyridine die allgemeine Formel (I)

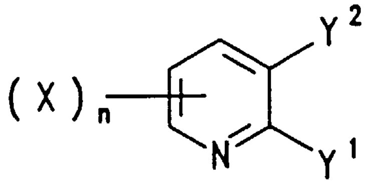

(I)

in der bedeuten

X = Halogen, vorzugsweise Fluor, Chlor oder Brom oder ein anderer elektronegativer Rest, wie eine Cyanogruppe oder ein Trifluormethylrest, vorzugsweise der Trifluormethylrest,
$Y^1/Y^2$ = unabhängig voneinander Fluor oder Chlor, wobei jedoch nicht beide Reste gleichzeitig Fluor bedeuten und
n = 1 oder 2, vorzugsweise 1.

Erfindungsgemäß werden insbesondere solche Ausgangsverbindungen (I) eingesetzt, bei denen der Rest X (bei n = 1) in der 5- oder 6-Position steht. Beispiele hierfür sind 2,3,5-Trichlorpyridin, 2,3,6-Trichlorpyridin, 2,3,5,6-Tetrachlorpyridin, 2,3-Dichlor-5-trifluormethylpyridin, 2,3-Dichlor-5-cyanopyridin, 3,5-Dichlor-2-fluorpyridin oder 2,5-Dichlor-3-fluorpyridin. Die Herstellung dieser Ausgangsverbindungen ist Stand der Technik. So kann beispielsweise das 2,3,5-Trichlorpyridin in bekannter Weise aus Chloral und Acrylnitril oder aus Trichloracetonitril und Acrolein unter Kupfer(I)-Katalyse (E. Steiner, P. Martin, D. Bellus, Helv. Chim. Acta, 65 (3) (1982), 983-985; SU-PS 1.583.416; SU-PS 1.182.035) hergestellt werden. Ein weiterer Syntheseweg besteht in der Chlorierung von 3,5-Dichlor-2-pyridon mittels Phosphoroxichlorid (JP-OS 54.059.283), wobei ersteres zum Beispiel entweder durch Chlorierung von 6-Hydroxynikotinsäure (EP-OS 206.293) oder in einem mehrstufigen Verfahren aus 2-Pyridon (JP-OS 01.075.468) oder aus 2-Aminopyridin durch Chlorierung und Diazoverkochung (JP-OS 54.059.283) oder einfache Chlorierung von 2-Pyridon (Cava, Bhattacharyya, J. Org. Chem., 23 (1958) 1614) zugänglich ist.

2,3,6-Trichlorpyridin erhält man beispielsweise bei der Chlorierung von 2,6-Dichlorpyridin (EP-PS 239 904), beim Behandeln von Pentachlorpyridin mit Hydrazin in Ethanol/Triethylamin und Natronlauge (US-PS 3 947 457), und selektiv bei der Reduktion von Pentachlorpyridin mit Lithiumaluminiumhydrid (F. Binns, S.M. Roberts, H. Suschitzky, J. Chem. Soc. C, (1970) (10), 1375-1380; J. Chem. Soc. D (1969) (20), 1211-1212). Auf diese Weise kann auch 2,3,5,6-Tetrachlorpyridin erhalten werden, sofern man weniger Reduktionsmittel einsetzt.

Das weiterhin als Ausgangsverbindung eingesetzte 2,3-Dichlor-5-trifluormethylpyridin kann hergestellt werden aus 2,2-Dichlor-3,3,3-trifluorpropionaldehyd und Acrylnitril unter Kupfer(I)katalyse (US-PS 4 469 896). Ebenso möglich ist die Umsetzung von 2,3-Dichlor-5-trichlormethyl-pyridin mit Fluorwasserstoff (EP-PS 110 690), wobei die Trichlormethylverbindung ihrerseits durch Chlorierung von 2-Chlor-5-trichlormethylpyridin mittels Metallcarbonylkatalysatoren hergestellt werden kann (US-PS 4 331 811).

Die nach dem erfindungsgemäßen Verfahren erhältlichen substituierten 2,3-Difluorpyridine haben vorzugsweise die allgemeine Formel (II)

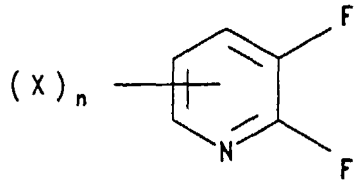

(II)

wobei X und n die Bedeutung gemäß obiger Formel (I) besitzen. Vorzugsweise handelt es sich dabei um 5-Chlor-2,3-difluorpyridin.

Diese Stoffe sind wichtige Vorprodukte für die Herstellung von Agrochemikalien und Pharmazeutika. So kann beispielsweise das 5-Chlor-2,3-difluorpyridin für die Herstellung von Pyridinyloxyphenoxyalkancarbonsäurederivaten dienen, die Herbizide mit besonders vortelihaften Eigenschaften darstellen (vgl. EP-Offenlegungsschriften 97.460; 142.328; 248.968 und 296.518 sowie US-Patentschriften 4.750.931 und 4.935.051). Auch das erfindungsgemäß erhältliche 2,3-Difluor-5-trifluormethylpyridin (vgl. such EP-OS 104 715) kann, wie das 5-Cyano-2,3-difluorpyridin, als Baustein für in Pflanzenschutzmitteln enthaltenen Wirkstoffen dienen (EP-OS 97 460).

Das 2,3,6-Trifluorpyridin, das erfindungsgemäß aus Verbindungen (I) mit X = Cl (hier 3-facher Chloraustausch) oder X = F (zweifacher Chloraustausch) und n = 1 darstellbar ist, kann als Vorstufe für antibakterielle Mittel der Naphthyridincarbonsäurereihe Verwendung finden (H. Koga, A. Itoh, S. Mursyama, S. Suzue, T. Irikura, J. Med. Chem. 23 (1980), 1358; H. Egawa, T. Miyamoto,

A. Minamida, Y. Nishimura, H. Okada, H. Uno, J. Matsumoto, J. Med. Chem. 27 (1984), 1543; EP-OS 153 828 und EP-OS 388 298).

Als Fluoridsalze kommen erfindungsgemäß vorzugsweise Kalium- oder Tetraalkyl-($C_1$-$C_{18}$)ammoniumfluorid oder deren Mischungen in Mischung mit Cäsiumfluorid in Frage, wobei diese Mischungen mit Cäsiumfluorid nicht mehr als 50 Gew.-% an Cäsiumfluorid enthalten. Bevorzugt werden Fluoridmischungen verwendet, die zumindest 75 Gew.-% Kaliumfluorid enthalten; insbesondere bestehen derartige Mischungen aus wenigstens 90 Gew.-% Kaliumfluorid und höchstens 10 Gew.-% Cäsiumfluorid. Eine erfindungsgemäße Mischung aus 90 Gew.-% Kaliumfluorid und 10 Gew.-% Cäsiumfluorid (siehe Beispiele) wird als "Kaliumfluorid/Cäsiumfluorid (9:1)" bezeichnet und eine Mischung aus 60 Gew.-% Kaliumfluorid und 40 Gew.-% Rubidiumfluorid heißt "Kaliumfluorid/Rubidiumfluorid (3:2)".

Pro auszutauschendes Chloratom werden etwa 0,9 bis etwa 5, bevorzugt zwischen etwa 1 und etwa 2 und besonders bevorzugt zwischen etwa 1 und etwa 1,5 Equivalente Fluoridsalz eingesetzt.

Die erfindungsgemäße Austauschreaktion wird vorzugsweise in polar aprotischen Lösungsmitteln durchgeführt. Als solche sind beispielsweise geeignet: Sulfolan (Tetramethylensulfon), Tetra-methylensulfoxid (TMSO), N,N-Diethylacetamid, N,N-Dimethylacetamid (DMAc), N,N-Dimethylformamid (DMF), N-Methylpyrrolidon (NMP), Dimethylsulfoxid (DMSO), Dimethylsulfon, Diphenylsulfoxid, Diphenylsulfon, Tetramethylharnstoff, Tetra-n-butylharnstoff, 1,3-Dimethylimidazolidin-2-on (DMI) oder Mischungen derartiger Lösungsmittel. Bevorzugt werden Tetramethylensulfon (Sulfolan), N,N-Dimethylacetamid (DMAc) und 1,3-Dimethylimidazolidin-2-on (DMI) verwendet; besonders bevorzugt sind Tetramethylensulfon (Sulfolan) und 1,3-Dimethylimidazolidin-2-on (DMI).

Das erfindungsgemäße Verfahren kann auch in anderen Lösungsmitteln durchgeführt werden. Dazu kommen unpolare Lösungsmittel, wie zum Beispiel Chlornaphthaline, Chlorbenzol, Di-chlorbenzole, Xylole oder Toluol in Frage. Auch die Durchführung in der Schmelze ohne Zusatz eines Lösungsmittels ist erfindungsgemäß möglich.

Als Phasentransferkatalysatoren können quartäre Ammonium- oder Phosphoniumsalze verwendet werden. An geeigneten Verbindungen seien folgende genannt: Tetraalkyl-($C_1$-$C_{18}$)-ammoniumchloride, -bromide oder -fluoride, Tetraalkyl-($C_1$-$C_{18}$)-phosphoniumchloride oder -bromide, Tetraphenylphosphoniumchlorid oder -bromid, (Phenyl)$_m$(alkyl($C_1$-$C_{18}$))$_n$-phosphoniumchloride oder -bromide, wobei m = 1 bis 3, n = 3 bis 1 und m + n = 4 ist. Auch Gemische dieser Salze sind einsetzbar. Bei geeigneter Wahl des Katalysators für die jeweilige umzusetzende Verbindung, was durch einige Routineversuche leicht zu ermitteln ist, erhält man deutlich bessere Raumleistungen und Ausbeuten als nach dem in der EP-OS 146 924 beschriebenen Verfahren; außerdem sind die Apparate-Totzeiten sowie die gesamten Apparatekosten nach dem erfindungsgemäßen Verfahren wesentlich günstiger.

Die Menge an Phasentransferkatalysator beträgt im allgemeinen bis zu 20 Gew.-%, bevorzugt zwischen 3 und 15 Gew.-% und besonders bevorzugt zwischen 3 bis 8 Gew.-%, bezogen auf die eingesetzte Menge an Fluoridsalz.

Als Phasentransferkatalysatoren können auch Oligo- oder Polyalkylenglykoldimethylether verwendet werden, wobei der Alkylenrest 2 bis 6 C-Atome, vorzugsweise 2 und/oder 3 C-Atome enthält, also vorzugsweise den Ethylen- und/oder den Propylenrest und insbesondere nur den Ethylenrest darstellt. Die Zahl der O-ethylen(Glykol)einheiten (-O-$CH_2$-$CH_2$-) und/oder der O-propyleneinheiten in diesen Verbindungen kann von n = 4 (z. B. Tetraethylenglykoldimethylether) bis etwa n = 150 betragen; bevorzugt werden jedoch Ether eingesetzt, deren Polymerisationsgrad zwischen n = 4 und n = 25 beträgt. Im Falle von Alkylenresten mit mehr als 3 C-Atomen liegt n im allgemeinen nicht höher als 6. Die Einsatzmenge dieser Ether, insbesondere Glykolether liegt zumeist zwischen etwa 0,5 Gew.-% und etwa 200 Gew.-%, bevorzugt zwischen etwa 5 und etwa 100 Gew.-% und besonders bevorzugt zwischen etwa 10 und etwa 50 Gew.-%, bezogen auf die Menge des eingesetzten Fluoridsalzes. Der besondere Vorteil bei der Verwendung dieser Verbindungen liegt darin, daß meist der Einsatzmenge entsprechend weniger Lösungsmittel verwendet werden kann, da die Glykolether bei der Reaktionstemperatur im allgemeinen flüssig wird. Auch Gemische dieser Ether untereinander als auch Gemische dieser Ether (einzeln oder im Gemisch) mit den quartären Ammonium- oder Phosphoniumsalzen, vorzugsweise Glykolether mit quartären Phosphoniumsalzen, können eingesetzt werden.

Verwendet man als Fluoridsalz Tetraalkyl($C_1$-$C_{18}$)ammoniumfluoride, so ist der Zusatz eines weiteren Phasentransferkatalysators nicht erforderlich, da das Fluoridsalz selbst einen solchen darstellt, der damit also in stöchiometrischen und größeren Mengen eingesetzt werden kann.

Die Temperaturen, bei denen die erfindungsgemäße Reaktion erfolgt, liegen im Bereich von etwa 120°C bis etwa 250°C, vorzugsweise von etwa 170°C bis etwa 230°C und besonders bevorzugt von etwa 190 bis 215°C. Wesentlich ist dabei, daß erfindungsgemäß das erhaltene Produkt nicht aus der Reaktionsmischung destillativ entfernt wird. Nach Beendigung der Austauschreaktion, die je nach angewandter Temperatur zumeist etwa 3 bis etwa 20 Stunden, vorzugsweise etwa 5 bis etwa 8 Stunden dauert, wird das gewünschte Austauschprodukt von dem nicht umgesetzten Ausgangsprodukt sowie von Nebenprodukten, wie z.B. dem Mono- sowie ggf. dem Trifluorierungsprodukt sowie etwaigen Isomerisierungsprodukten, abgetrennt. Dies geschieht in der Regel durch fraktionierte Destillation, wobei das Reaktionssalz im allgemeinen zunächst durch Filtration abgetrennt, danach der Filterkuchen mit einem organischen Lösungsmittel gewaschen und anschließend die Mutterlauge fraktioniert wird. Ebenfalls praktikabel ist, direkt aus der Reaktionsmischung die leichtflüchtigen Bestandteile abzudestillieren, um das erhaltene Robdestillat dann anschließend einer Feinfraktionierung zu unterwerfen. Das Lösungsmittel wird bei dieser Verfahrensweise getrennt recyclisiert. Daneben ist auch eine wäßrige Aufarbeitung möglich, wobei das Produktgemisch durch Extraktion aus der Wasserphase gewonnen und durch chromatographische Methoden gereinigt wird. Als Extraktionsmittel eignen sich besonders unpolare organische Lösungsmittel, wie zum Beispiel Cyclohexan oder Xylol (siehe auch Beispiele). Weiterhin kann auch die Schmelzkristallisation zur Reinigung eingesetzt werden.

Das erfindungsgemäße Verfahren kann bei Einsatz von Di(Tri)chlorverbindungen einstufig oder auch zweistufig durchgeführt werden. Im letzteren Falle wird die betreffende Di(Tri)chlorverbindung, beispielsweise 2,3,5-Trichlorpyridin, zunächst zur entsprechenden Monofluorverbindung umgesetzt und die dabei erhaltene Reaktionsmischung ohne Isolierung der Monofluorverbindung dann anschließend in einer zweiten Stufe mit weiterem Fluoridsalz und, ggfs. bei gegenüber der ersten Stufe erhöhter Temperatur, zur gewünschten Di(Tri)fluorverbindung umgesetzt.

Das erfindungsgemäße Verfahren erweist sich als besonders vorteilhaft, da verfahrenstechnische Schwierigkeiten bel der technischen Realisierung, wie sie bei alternativen Herstellverfahren beispielsweise für 5-Chlor-2,3-difluorpyridin auftreten, umgangen werden können. Hier sind besonders Werkstoffprobleme und aufwendige Apparatetechnik zu nennen. Es wird durch dieses direkte Verfahren außerdem die Vielstufigkeit der Synthese vermieden. Die Verwendung von sprühgetrocknetem Alkalimetallfluorid im erfindungsgemäßen Verfahren verkürzt dabei zwar zum Teil die Reaktionszeiten, ist aber nicht unbedingt notwendig. Ebenso kann unter Zusatz von säurebindenden Mittein, wie Alkali- und Erdalkalimetallcarbonaten oder basisch wirkenden Oxiden, wie zum Beispiel Magnesiumoxid, oder entsprechenden Gemischen, gearbeitet werden. Besonders bevorzugt ist hierbei Kaliumcarbonat, das in Anteilen von etwa 1 bis etwa 10 Gew.-%, bevorzugt von etwa 4 bis etwa 6 Gew.-%, bezogen auf die Fluoridsalz-Menge, verwendet wird. Die säurebindenden Mittel sind für den Reaktionsverlauf im allgemeinen nicht wesentlich. In einigen Fällen wird die Reaktionsgeschwindigkeit durch die Bildung von Fluorwasserstoff während der Reaktion erheblich verringert. In diesen Fällen ist es günstig, besonders auch zur Vermeidung von Apparatekorrosion, unter Anwesenheit von derartigen Säurefängern zu arbeiten. Der Einsatz dieser Verbindungen bei Fraktionierung des Reaktionsgemisches oder des Rohproduktes kann aus Gründen der Korrosion in der Fraktionieranlage wünschenswert sein, wobei Magnesiumoxid hierbei besonders bevorzugt ist. Zu diesem Zweck werden der Fraktionierblase bis etwa 10 Gew.-% an Säurefänger zugesetzt, bevorzugt zwischen etwa 3 und 8 Gew.-%, bezogen auf die Gesamtmenge an eingesetztem Destillationssumpf.

Das erfindungsgemäße Verfahren kann bei Unter-, Über- oder Atmosphärendruck durchgeführt werden. Bevorzugt ist, bei Überdruck zu arbeiten, um ein Sieden der Reaktionskomponenten zu vermeiden, da dies unter Umständen zu aus wirtschaftlichen und arbeitshygienischen Gründen nicht wünschenswerten Verlusten führen kann.

Das erfindungsgemäße Verfahren besitzt gegenüber bekannten Verfahren, besonders gegenüber Verfahren, die unter Abdestillieren des Produktes arbeiten, wesentliche, überraschende Vorteile. Um das gleichmäßige Abdestillieren des Produktes ohne wesentliche Mengen an Zwischenprodukten, die noch weiter umgesetzt werden müssen, zu gewährleisten, wird bei den bekannten Verfahren eine aufwendige Steuerungs- und Regelungstechnik notwendig, jedoch ist trotz dieser stets noch eine darauffolgende Reinfraktionierung notwendig. Die dafür primär benötigten Destillationsaufsätze auf Reaktionsapparaten sind im allgemeinen in installierten Anlagen nicht vorgesehen. Zudem können beim Abdestillieren unter Reaktionsbedingungen Hygieneprobleme durch teilweise nicht zu kondensierende, leichtsiedende Bestandteile auftreten, sofern die Steuer- und Regelungseinheiten nicht vollständig zuverlässig funktionieren, was bei der behandelten Substanzklasse aber von hoher Wichtigkeit ist. Bei nachträglicher Destillation, wie beim erfindungsgemäßen Verfahren, kann bereits durch Destillation der rohen Destillationsmischung genügende Reinheit des Produktes und der in die nächste Charge zurückführenden Anteile erreicht werden, sodaß sich eine insgesamt wesentlich einfachere und besonders wegen nur einfacher Destillation kostengünstige Aufarbeitung ergibt. Um die Rührbarkeit der Reaktionsmischungen zu gewährleisten, sind hohe Mengen an Lösungsmittel notwendig, da ein Großteil der flüssigen Komponenten während der Umsetzung aus der Mischung entfernt wird und Salze sowie meist harzige Nebenprodukte zurückbleiben. Das erfindungsgemäße Verfahren besitzt dadurch weiterhin den Vorteil, daß unter Verwendung geringer Lösemittelmengen bzw. ohne Lösungsmittel in der Schmelze gearbeitet werden kann, wodurch die Raumleistungen und somit die Kapazität der Anlagen wesentlich erhöht werden kann. Durch die Verwendung der erfindungsgemäßen

Phasentransferkatalysatoren gelingt außerdem die weitgehende Umsetzung ohne Einsatz von hohen Mengen an teurem Cäsiumfluorid oder von Dimethylsulfoxid und ohne erhebliche Zersetzung, wie dies bei literaturbekannten Verfahren bekannt ist. Durch die deutlich geringeren Zersetzungsanteile ist die Menge an harzigen und damit unbekannten Nebenprodukten stark rückläufig. Dadurch werden neben ökonomischen auch ökologische Vorteile erzielt, da weniger Material verbrannt oder deponiert werden muß. Auch besteht die einfachere Möglichkeit zur Wiederaufarbeitung der ausreagierten Reaktionssalze, da diese mit weniger organischen Verunreinigungen behaftet sind. Bei stark verunreinigten Salzen, wie diese aus bekannten Verfahren anfallen (z.B. EP 146 924), wurde die Aufarbeitung und Weiterverwendung der Salze je nach Charge unmöglich. Wenn Lösungsmittel aus technischen Gründen im erfindungsgemäßen Verfahren verwendet werden, so ist in keinem Fall die Verwendung von Dimethylsulfoxid notwendig. Dieses ist wegen der hohen Toxizität, Hautgängigkeit und hohen Geruchsbelästigung, besonders bei thermischer Belastung, in der technischen Anwendung nur noch in Ausnahmefällen verwendbar.

Die folgenden Beispiele sollen das erfindungsgemäße Verfahren erläutern, ohne es darauf zu beschränken.

Ausführungsbeispiele

In einigen Beispielen wurde der Reaktionsverlauf durch Eichung mittels internem Standard (inert unter den Reaktionsbedingungen) gaschromatograpisch verfolgt. Dadurch läßt sich zu beliebigen Zeitpunkten exakt die gebildete Menge an Produkten bestimmen, die durch Aufarbeitung erhalten werden kann. Diese Ansätze könnten problemlos ohne Zusatz des inneren Standards in größerem Maßstab mit anschließender Aufarbeitung durchgeführt werden, wobei sogar im allgemeinen durch Verbesserung der Rührbedingungen bessere Ergebnisse wie kürzere Reaktionszeiten, höhere Raumleistungen und bessere Ausbeuten erhalten werden.

Die Aufarbeitung der Reaktionsmischungen erfolgte im allgemeinen durch Filtration vom Reaktionssalz, Waschen des Filterkuchens mit einem organischen Lösungsmittel und anschließender Fraktionierung der Mutterlauge. Ebenfalls praktikabel ist, direkt aus der Reaktionsmischung die leichtflüchtigen Bestandteile abzudestillieren, um das erhaltene Rohdestillat dann anschließend einer Feinfraktionierung zu unterwerfen. Das Lösungsmittel wird bei dieser Verfahrensweise getrennt recyclisiert.

Beispiel 1 (Vergleichsbeispiel)

In einem 100 ml-Dreihalskolben mit günstiger Rührung (Stromstörelemente), Innentemperatursteuerung und Intensivkühler wurden 18,2 g (0,1 Mol) 2,3,5-Trichlorpyridin zu einer Suspension von 13,9 g (0,24 Mol; 20 % Überschuß) Kaliumfluorid, 0,5 g Kaliumcarbonat und 1 g Pentamethylbenzol (innerer Standard) in 50 g Sulfolan gegeben, wobei die Mischung vorher durch azeotrope Destillation (3 Torr, 140°C) getrocknet worden war. Die Mischung wurde 24 h auf 200°C erhitzt (Rückfluß), die Zusammensetzung war nach dieser Zeit wie folgend angegeben:

2,3,5-Trichlorpyridin: 11 % d.Th.
5-Chlor-2,3-difluorpyridin: 5 % d.Th (Raumleistung: 0,5 g/l • h; Selektivität: 14%).
3,5-Dichlor-2-fluorpyridin: 38 % d.Th.
2,3,5-Trifluorpyridin: < 1 % d.Th.

Die angegebenen Ausbeuten wurden durch Eichung gegen den inneren Standard bestimmt.

Arbeitete man unter ständigem Abdestillieren, so erhielt man insgesamt 18 % d. Th. 5-Chlor-2,3-difluorpyridin (Raumleistung: 2,0 g/l • h; Selektivität: 40 %), 30 % d. Th. 3,5-Dichlor-2-fluorpyridin und 10 % 2,3,5-Trichlorpyridin.

Setzte man reines Cäsiumfluorid (0,28 Mol; 40 % Überschuß) und DMSO als Lösungsmittel ein (wie in EP-OS 146 924, Beispiel 11 beschrieben) und arbeitete unter ständigem Abdestillieren, so isolierte man 50,4 % d. Th. 5-Chlor-2,3-difluorpyridin (Raumleistung: 5,2 g/l • h; Selektivität: 67 %) und 7,8 % d. Th. 3,5-Dichlor-2-fluorpyridin.

Beispiel 2

Es wurde wie im Beispiel 1 verfahren, jedoch wurden 14,9 g (0,24 Mol) Kaliumfluorid/Cäsiumfluorid (9:1) anstatt von Kaliumfluorid und 1,4 g (10 Gew.-%, bezogen auf Fluoridmischung) Tetraphenylphosphoniumbromid als Phasentransferkatalysator eingesetzt, das ebenfalls der azeotropen Trocknung unterworfen wurde. Die Mischung wurde 14 h auf 215°C erhitzt (Rückfluß); die Zusammensetzung war nach dieser Zeit wie folgend angegeben:

2,3,5-Trichlorpyridin: 2 % d.Th.
5-Chlor-2,3-difluorpyridin: 14 % d.Th (Raumleistung: 3,2 g/l • h Selektivität: 39%).
3,5-Dichlor-2-fluorpyridin: 45 % d.Th.
2,3,5-Trifluorpyridin: < 1 % d.Th.

Die angegebenen Ausbeuten wurden durch Eichung gegen den inneren Standard bestimmt.

Beispiel 3

Es wurde wie im Beispiel 2 gearbeitet; als Fluoridquelle wurden 15,3 g (0,24 Mol) Kaliumfluorid/Cäsiumfluorid (6:1) und als Phasentransferkatalysator 1,5 g Tetrabutylphosphoniumbromid eingesetzt. Nach einer Reaktionszeit von 9 h bei 215°C hatte die Reaktionsmischung die folgende Zusammensetzung:

2,3,5-Trichlorpyridin: < 1 % d.Th.
5-Chlor-2,3-difluorpyridin: 34 % d.Th (Raumleistung: 12,0 g/l • h; Selektivität: 87%).
3,5-Dichlor-2-fluorpyridin: 43 % d.Th.
2,3,5-Trifluorpyridin: 2 % d.Th.

Die angegebenen Ausbeuten wurden durch Eichung gegen den inneren Standard bestimmt.

Beispiel 4

Analog Beispiel 2 wurden 14,6 g (0,24 mol) Kaliumfluorid/Cäsiumfluorid (12:1) und 1,5 g Ethyltrioctylphosphoniumbromid als Phasentransferkatalysator verwendet. Die Reaktionsmischung wurde 9 h bei 215°C gehalten und danach durch GC (innerer Standard) analysiert:

2,3,5-Trichlorpyridin: ca. 1 % d.Th.
5-Chlor-2,3-difluorpyridin: 39 % d.Th. (Raumleistung: 13,9 g/l • h; Selektivität: 92,5%).
3,5-Dichlor-2-fluorpyridin: 40 % d.Th.
2,3,5-Trifluorpyridin: 1 % d.Th.

Die angegebenen Ausbeuten wurden durch Eichung gegen den inneren Standard bestimmt.

Beispiel 5

Gemäß der Arbeitsweise nach Beispiel 2 wurden 1,5 g Tetraethylenglykol-dimethylether und 14,9 g (0,24 Mol) Kaliumfluorid/Cäsiumfluorid (9:1) eingesetzt; die Reaktionsmischung wurde 15 h bei 215°C gehalten. Deren Zusammensetzung ergab sich nach dieser Zeit wie folgt:

2,3,5-Trichlorpyridin: < 1 % d.Th.
5-Chlor-2,3-difluorpyridin: 24 % d.Th (Raumleistung: 5,1 g/l • h; Selektivität: 70%).
3,5-Dichlor-2-fluorpyridin: 48 % d.Th.
2,3,5-Trifluorpyridin: 4 % d.Th.

Die angegebenen Ausbeuten wurden durch Eichung gegen den inneren Standard bestimmt.

Beispiel 6 (zweistufiges Verfahren)

Zunächst wurden 18,2 g (0,1 Mol) 2,3,5-Trichlorpyridin in 50 g Sulfolan unter Anwesenheit von 1,4 g Tetra-n-octylphosphoniumbromid als Phasentransferkatalysator 5 h bei 200°C mit 7,0 g (0,12 Mol) Kaliumfluorid umgesetzt. Nach dieser Zeit befanden sich in der Mischung 73 % d.Th. an 3,5-Dichlor-2-fluorpyridin (Selektivität: 83 %) und 12 % d.Th. an 2,3,5-Trichlorpyridin. Das nach dem Abkühlen vorhandene Salz wurde abgesaugt und mit 10 g warmem Sulfolan (azeotrop getrocknet) gewaschen. Die Muttertauge wurde anschließend mit 7,8 g (0,12 Mol) scharf getrocknetem Kaliumfluorid/Cäsiumfluorid (5:1) versetzt und auf 215°C geheizt. Die Mischung hatte nach weiteren 5 h folgende Zusammensetzung (GC, innerer Standard):

2,3,5-Trichlorpyridin: ca. 2 % d.Th.
5-Chlor-2,3-difluorpyridin: 42 % d.Th (Raumleistung: 10,5 g/l • h; Selektivität: 72%).
3,5-Dichlor-2-fluorpyridin: 23 % d.Th.
2,3,5-Trifluorpyridin: < 1 % d.Th.

Die angegebenen Ausbeuten wurden durch Eichung gegen den inneren Standard bestimmt.

Beispiel 7

73,0 g (0,4 Mol) 2,3,5-Trichlorpyridin wurden zu einer Suspension von 59,4 g (0,96 Mol) Kaliumfluorid/Cäsiumfluorid (9:1), 5,9 g Oktadecyltrimethylammoniumchlorid, 2,0 g Kaliumcarbonat und 4 g Pentamethylbenzol (innerer Standard) in 50 g N,N-Dimethylacetamid gegeben, wobei die Mischung vorher durch azeotrope Destillation (3 Torr, 140°C) getrocknet worden war. Die Mischung wurde 11 h in einem Glasautoklaven auf 190°C erhitzt (Rückfluß), die Zusammensetzung war nach dieser Zeit wie folgend angegeben:

2,3,5-Trichlorpyridin: < 1 % d.Th.
5-Chlor-2,3-difluorpyridin: 25 % d.Th (Raumleistung: 11,3 g/l • h; Selektivität: 51%).
3,5-Dichlor-2-fluorpyridin: 33 % d.Th.
2,3,5-Trifluorpyridin: ca. 1 % d.Th.

Die angegebenen Ausbeuten wurden durch Eichung gegen den inneren Standard bestimmt.

Beispiel 8

850 g Sulfolan, 204,3 g (3,3 Mol) Kaliumfluorid/Cäsiumfluorid (9:1), 7,5 g Kaliumcarbonat und 20,4 g Tetra-n-octylphosphoniumbromid als Phasentransferkatalysator wurden durch Abdestillieren von ca. 100 g Sulfolan (170°C) azeotrop getrocknet. Bei 190°C wurden anschileßend 273,5 g (1,5 Mol) 2,3,5-Trichlorpyridin zugegeben und die Reaktionsmischung auf 215°C geheizt. Nach etwa 2 h setzte Rückfluß ein. Dieser wurde 20 h aufrechterhalten, und anschließend wurde aus der Reaktionsmischung sämtliche Substanz abdestilliert, die unterhalb von 3 Torr/140°C siedete. Man erhielt eine erste Fraktion (15,8 g), die 14,2 g 5-Chlor-2,3-difluorpyridin und 1,3 g 3,5-Dichlor-2-fluorpyridin enthielt (durch GC mittels innerem Standard ermittelt). Die zweite Fraktion (168.0 g) enthielt 89,4 g 5-Chlor-2,3-difluorpyridin und 64,5 g 3,5-Dichlor-2-fluorpyridin, die dritte Fraktion (Sdp. > 130°C/4 Torr) enthielt 98,5 g (GC) Sulfolan. Die Fraktionen wurden durch Fraktionierung gereinigt. Die Ausbeute betrug 46 % für das 5-Chlor-2,3-difluorpyridin und 27 % für das 3,5-Dichlor-2-fluorpyridin (Summe 73 %, isoliert). Die Ausbeute an 5-Chlor-2,3-difluorpyridin war 63 %, bezogen auf umgesetztes Material. Nicht berücksichtigt wurde hierbei jeweils der Anteil an 3-Chlor-2,5-difluorpyridin, der zusätzlich Ca. 5 % der Menge an 5-Chlor-2,3-difluorpyridin betrug. (Raumleistung bezüglich 5-Chlor-2,3-difluor-pyridin: 4,7 g/l • h; Selektivität: 82 %.)

Beispiel 9

36,4 g (0,2 Mol) 2,3,6-Trichlorpyridin und 1 g Pentamethylbenzol wurden zu einer durch azeotrope Destillation getrockneten Suspension von 18 g (0,3 Mol) Kaliumfluorid/Cäsiumfluorid (9:1), 2,5 g Tetra-n-butylphosphoniumbromid und 1 g Kaliumcarbonat in 150 g Sulfolan gegeben. Man erhitzte die Mischung 14 h auf 215°C in einem Glasautoklaven und erhielt die folgende Mischung:

2,3,6-Trifluorpyridin: 38 % d.Th.
3-Chlor-2,6-difluorpyridin: 23 % d.Th.
3,6-Dichlor-2-fluorpyridin: ca. 2 % d.Th.

Die angegebenen Ausbeuten wurden durch Eichung gegen den inneren Standard bestimmt.

Beispiel 10:

86,4 g (0,4 Mol) 2,3-Dichlor-5-trifluormethylpyridin wurden zu einer azeotrop getrockneten Suspension aus 50,3 g (0,84 Mol) Kaliumfluorid/Cäsiumfluorid (20:1), 1,5 g Hexadecyltriethylphosphoniumbromid und 1 g Kaliumcarbonat in 100 g 1,3-Dimethylimidazolidin-2-on gegeben (innerer Standard 1 g Pentamethylbenzol). Die Mischung wurde in einem Glasautoklaven für 12 h auf 190°C erhitzt und anschließend durch GC analysiert. In der Mischung waren 51,8 g (0,28 Mol/71 % d. Th.) 2,3-Difluor-5-trifluormethylpyridin und 5 g (25 mMol, 6 % d. Th.) 3-Chlor-2-fluor-5-trifluormethylpyridin enthalten. Diese konnten durch Destillation aus dem Gemisch abgetrennt und danach durch Fraktionierung gereinigt werden. Der Siedepunkt des Produkts betrug 116 - 117°C bei 760 Torr, das Zwischenprodukt siedete zwischen 137°C und 140°C.

Sdp. 74 (66)°C/125 (108) mbar (5-Chlor-2,3-difluorpyridin)
Sdp. 96°C/79 mbar (3,5-Dichlor-2-fluorpyridin)
Sdp. ca. 142°C/1013 mbar (3-Chlor-2,5-difluorpyridin)

5-Chlor-2,3-difluorpyridin:

$^1$H-NMR ($CDCl_3$, interner Standard TMS):

δ = 7,61 (ddd,1H, J=2,24 Hz (H-6), 7,89 Hz (F-2), 8,41 Hz (F-3), H-4) 7,97 (dd, 1H, J = 2,24 Hz (H-4), 1,80 Hz (F-2), H-6)

$^{19}$F-NMR ($CDCl_3$, interner Standard $CFCl_3$):

δ = -88,80 (ddd,1F,J=1,80 Hz (H-6), 7,89 Hz (H-4), 25,67 Hz (F-3), F-2) -136,51 (dd, 1F, J=8,41 Hz (H-4), 25,67 Hz (F-2), F-3)

MS: m/z (%) = 50 (4), 64 (25), 69 (7), 87 (8), 94 (4), 114 (63), 122 (7), 130 (1), 149 ($M^+$, 100), 151 (34)

3-Chlor-2,5-difluorpyridin:

$^{19}$F-NMR ($CDCl_3$, interner Standard $CFCl_3$):

δ = -75,63 (d, 1F, J = 28,0 Hz (F-5), F-2) -129,9 (dd, 1F, J = 6,4 Hz (H-4), 28,0 Hz (F-2), F-5)

3,5-Dichlor-2-fluorpyridin:

$^1$H-NMR ($CDCl_3$, interner Standard TMS):

δ = 7,83 (dd, 1H, J = 2,32 Hz (H-6), 7,63 Hz (F-2), H-4) 8,07 (dd, 1H, J = 2,32 Hz (H-4), 1,47 Hz (F-2), H-6)

$^{19}$F-NMR ($CDCl_3$, interner Standard $CFCl_3$):

δ = -73,77 (dd, 1F, J = 1,47 Hz (H-6), 7,63 Hz (H-4), F-2)

MS: m/z (%) = 50 (8), 68 (14), 75 (5), 85 (6), 94 (8), 103 (13), 110 (13), 130 (63), 165 ($M^+$, 100), 167 (67), 169 (12)

Beispiel 11

In 990 g Sulfolan wurden 254,7 g (4,38 Mol) Kaliumfluorid in Mischung mit 45,0 g (0,296 Mol) Cäsiumfluorid und 5,7 g Kaliumcarbonat suspendiert und von der Suspension 90 g Sulfolan im Vakuum abdestilliert. Zu der verbleibenden Mischung gab man 28,4 g (50,4 mMol) Tetra-n-octylphosphoniumbromid und 328,4 g (1,8 Mol) 2,3,5-Trichlorpyridin. Man erhitzte die Mischung unter Rühren in einem Autoklaven (Hastelloy C4) 6 h auf 205°C, der resultierende Druck blieb unterhalb 0,5 bar. Nach dem Abkühlen wurden die leichtsiedenden Bestandteile bis zum reinen Sulfolan aus der Reaktionsmischung abdestilliert und quantitativ durch GC gegen inneren Standard (p-Xylol) untersucht. Man erhielt in der Mischung der abdestillierten leichtersiedenden Anteile (516,2 g) 144,5 g (0,967 Mol, 53,7 % d. Th.) 5-Chlor-2,3-difluorpyridin und 70,2 g (0,423 Mol, 23,5 % d. Th.) 3,5-Dichlor-2-fluorpyridin (Raumleistung: 20,1 g/l • h, Selektivität: 90 %).

Beispiel 12

Man gab 400 g Sulfolan, 129 g Kaliumcarbonat und 85,4 g Kaliumfluorid/Cäsiumfluorid (9:1) zusammen und destillierte bei 170°C/30 Torr 100 g Sulfolan ab. Zu der Mischung wurden 109,2 g (0,6 Mol 2,3,5-Trichlorpyridin und 6,8 g (12 mMol) Tetra-n-octylphosphoniumbromid gegeben und 6 h auf 200°C erhitzt. Von der Mischung wurden die leichtsiedenden Bestandteile abdestilliert und das Destillat analysiert (GC, quantitativ). Es waren 40,6 g (0,272 Mol, 45,3 % d. Th.) 5-Chlor-2,3-difluor-pyridin und 34,7 g (0,209 Mol, 34,8 % d. Th.) 3,5-Dichlor-2-fluorpyridin enthalten (Raumleistung: 16,1 g/l • h, Selektivität: 94 %). Zur Aufarbeitung des verbleibenden Sumpfes wurde wie in Beispiel 12 verfahren.

Beispiel 13 (Vergleichsbeispiel)

Man setzte in 300 g Sulfolan 109,2 g (0,6 Mol) 2,3,5-Trichlorpyridin mit 104,6 g (1,8 Mol) Kaliumfluorid unter Anwesenheit von 1,9 g Kaliumcarbonat 6 h bei 205°C um. Die Reaktionsmischung war, wie in den vorherigen Beispielen beschrieben, durch azeotropes Abdestillieren getrocknet worden. Nach Abdestillieren der leichtsiedenden Anteile konnten im Destillat (227 g) lediglich 5,1 g (39,2 mMol, 5,7 % d. Th.) nachgewiesen werden. 3,5-Dichlor-2-fluorpyridin war als Hauptbestandteil (82,7 g, 0,499 Mol, 83,1 %) enthalten (Raumleistung: 2,0 g/l • h, Selektivität: > 95 %).

**Patentansprüche**

1. Verfahren zur Herstellung von substituierten 2,3-Difluorpyridinen in der Schmelze oder in Anwesenheit eines polar aprotischen Lösungsmittels oder eines unpolaren Lösungsmittels, mit Ausnahme von Benzonitril, dadurch gekennzeichnet, daß man substituierte 2,3-Dihalogenpyridine aus der Gruppe substituierte 2,3-Dichlorpyridine, substituierte 2-Chlor-3-fluorpyridine oder substituierte 3-Chlor-2-fluorpyridine mit Kalium-, Rubidium- oder Tetraalkyl ($C_1$-$C_{18}$)ammoniumfluorid oder deren Mischungen in Mischung mit Cäsiumfluorid, wobei der Anteil an Cäsiumfluorid nicht mehr als 50 Gew.-% beträgt, in Anwesenheit von Phasentransferkatalysatoren bei Temperaturen zwischen 120°C und 250°C umsetzt, ohne die erhaltenen substituierten 2,3-Difluorpyridine ständig aus der Reaktionsmischung zu entfemen.

2. Verfahren nach Anspruch 1, dadurch gekennzeichnet, daß die Umsetzung in polar aprotischen Lösungsmitteln erfolgt.

3. Verfahren nach Anspruch 1 oder 2, dadurch gekennzeichnet, daß die substituierten 2,3-Halogenpyridine die allgemeine Formel (I)

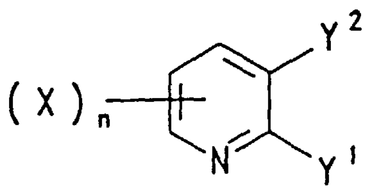

(I)

aufweisen, in der bedeuten

| | |
|---|---|
| X = | Halogen, vorzugsweise Chlor oder Brom, oder ein anderer elektronegativer Rest, vorzugsweise der Trifluormethylrest; |
| $Y^1/Y^2$ = | unabhängig voneinander Fluor oder Chlor, wobei jedoch nicht beide Reste gleichzeitig Fluor bedeuten; |
| n = | 1 oder 2, vorzugsweise 1. |

4. Verfahren nach Anspruch 3, dadurch gekennzeichnet, daß der Rest X (bei n = 1) in der 5- oder 6-Position steht.

5. Verfahren nach Anspruch 3 oder 4, dadurch gekennzeichnet, daß man als Verbindungen (I) 2,3,5-Trichlorpyridin, 2,3,6-Trichlorpyridin, 2,3,5,6-Tetrachlorpyridin, 2,3-Dichlor-5-trifluormethylpyridin, 2,3-Dichlor-5-cyanopyridin, 3,5-Dichlor-2-fluorpyridin oder 2,5-Dichlor-3-fluorpyridin einsetzt.

6. Verfahren nach einem oder mehreren der Ansprüche 1 bis 5, dadurch gekennzeichnet, daß als Fluoridsalze Kalium- oder Tetraalkyl-($C_1$-$C_{18}$)ammoniumfluorid oder Mischungen untereinander oder mit Cäsiumfluorid verwendet werden, wobei der Anteil an Cäsiumfluorid in derartigen Mischungen nicht mehr als 50 Gew.-% beträgt.

7. Verfahren nach einem oder mehreren der Ansprüche 1 bis 6, dadurch gekennzeichnet, daß man als Fluoridsalze Gemische von Alkalimetallfluoriden verwendet, die mindestens 90 Gew.-% Kaliumfluorid enthalten.

8. Verfahren nach Anspruch 7, dadurch gekennzeichnet, daß die Gemische aus mindestens 90 Gew.-% Kaliumfluorid und höchstens 10 Gew.-% Cäsiumfluorid bestehen.

9. Verfahren nach einem oder mehreren der Ansprüche 1 bis 8, dadurch gekennzeichnet, daß das Fluoridsalz in einer Menge entsprechend 0,9 bis 5 Equivalenten, vorzugsweise 1 bis 2 Equivalenten, pro auszutauschendes Chloratom eingesetzt wird.

10. Verfahren nach einem oder mehreren der Ansprüche 1 bis 9, dadurch gekennzeichnet, daß als Phasentransferkatalysatoren quartäre Ammonium- oder Phosphoniumverbindungen aus der Gruppe Tetraalkyl-($C_1$-$C_{18}$)-ammoniumchloride, -bromide oder -fluoride, Tetraalkyl-($C_1$-$C_{18}$)-phosphoniumchloride oder -bromide, Tetraphenylphosphoniumchlorid oder -bromid, $(Phenyl)_m(alkyl(C_1$-$C_{18}))_n$-phosphoniumchloride oder -bromide, wobei m = 1 bis 3, n = 3 bis 1 und m + n = 4 ist, verwendet werden.

**11.** Verfahren nach Anspruch 10, dadurch gekennzeichnet, daß man die Phasentransferkatalysatoren in Mengen bis zu 20 Gew.-%, bevorzugt zwischen 3 und 15 Gew.-%, bezogen auf die Menge an Alkalimetallfluorid, einsetzt.

**12.** Verfahren nach einem oder mehreren der Ansprüche 1 bis 9, dadurch gekennzeichnet, daß man als Phasentransferkatalysatoren Oligo- oder Polyethylenglykoldimethylether, die 4 bis 150 Glykoleinheiten ($-O-CH_2-CH_2-$) enthalten oder Gemische davon mit den Phasentransferkatalysatoren gemäß Anspruch 10 einsetzt.

**13.** Verfahren nach Anspruch 12, dadurch gekennzeichnet, daß man die Oligo- oder Polyethylenglykoldimethylether in Mengen von 0,5 bis 200 Gew.-%, vorzugsweise 5 bis 100 Gew.-%, bezogen auf die Menge an Fluoridsalz, verwendet.

**14.** Verfahren nach Anspruch 2, dadurch gekennzeichnet, daß als polar aprotische Lösungsmittel Sulfolan (Tetramethylensulfon), Tetramethylensulfoxid (TMSO), N,N-Diethylacetamid, N,N-Dimethylacetamid (DMAc), N,N-Dimethylformamid (DMF),
N-Methylpyrrolidon (NMP), Dimethylsulfoxid (DMSO), Dimethylsulfon, Diphenylsulfoxid, Diphenylsulfon, Tetramethylharnstoff, Tetra-n-butylharnstoff, 1,3-Dimethylimidazolidin-2-on (DMI) oder Mischungen derselben verwendet werden.

**15.** Verfahren nach einem oder mehreren der Ansprüche 1 bis 14, dadurch gekennzeichnet, daß man die Umsetzung bei Temperaturen zwischen 170°C und 230°C durchführt.

**16.** Verfahren nach einem oder mehreren der Ansprüche 1 bis 15, dadurch gekennzeichnet, daß man unter Anwesenheit von säurebindenden Mitteln, bevorzugt Alkali- oder Erdalkalimetallcarbonaten oder basisch wirkenden Oxiden arbeitet.

**17.** Verfahren nach Anspruch 16, dadurch gekennzeichnet, daß der Einsatz der säurebindenden Mittel bel der Fraktionierung erfolgt.

**18.** Verfahren nach Anspruch 16 oder 17, dadurch gekennzeichnet, daß man als säurebindende Mittel Natrium- und/oder Kaliumcarbonat und/oder Magnesiumoxid einsetzt.

**19.** Verfahren nach einem oder mehreren der Ansprüche 1 bis 18, dadurch gekennzeichnet, daß man unter Überdruck arbeitet.

**20.** Verfahren nach einem oder mehreren der Ansprüche 1 bis 19, dadurch gekennzeichnet, daß man als substituierte 2,3-Dihalogenpyridine substituierte 2,3-Dichlorpyridine einsetzt.

**21.** Verfahren nach einem oder mehreren der Ansprüche 1 bis 20, dadurch gekennzeichnet, daß man in einem zweistufigen Verfahren ein substituiertes 2,3-Dichlorpyridin zunächst zum substituierten 2-Fluor-3-chlorpyridin umsetzt und die dabei erhaltene Rohmischung erneut mit Fluoridsalz zum gewünschten 2,3-Difluorpyridin umsetzt

## Claims

**1.** A process for the preparation of substituted 2,3-difluoropyridines in the melt or in the presence of a polar aprotic solvent or a nonpolar solvent, with the exception of benzonitrile, which comprises reacting substituted 2,3-dihalopyridines from the group comprising substituted 2,3-dichloropyridines, substituted 2-chloro-3-fluoropyridines and substituted 3-chloro-2-fluoropyridines with potassium fluoride, rubidium fluoride or tetraalkyl($C_1$-$C_{18}$)ammonium fluoride or mixtures thereof mixed with cesium fluoride, the proportion of cesium fluoride being not more than 50% by weight, in the presence of phase transfer catalysts at temperatures between 120°C and 250°C, without removing the substituted 2,3-difluoropyridines obtained in this process continuously from the reaction mixture.

**2.** The process as claimed in claim 1, wherein the reaction is carried out in polar aprotic solvents.

**3.** The process as claimed in claim 1 or 2, wherein the substituted 2,3-halopyridines have the formula (I)

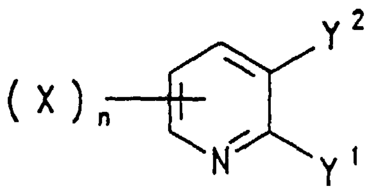

( I ) (I)

in which

X is halogen, preferably chlorine or bromine, or another electronegative radical, preferably, the trifluoromethyl radical;
$Y^1/Y^2$ independently of one another are fluorine or chlorine, where, however, both radicals are not simultaneously fluorine and
n is 1 or 2, preferably 1.

**4.** The process as claimed in claim 3, wherein the radical X (if n = 1) is in the 5- or 6-position.

**5.** The process as claimed in claim 3 or 4, wherein the compounds (I) employed are 2,3,5-trichloropyridine, 2,3,6-trichloropyridine, 2,3,5,6-tetrachloropyridine, 2,3-dichloro-5-trifluoromethylpyridine, 2,3-dichloro-5-cyanopyridine, 3,5-dichloro-2-fluoropyridine or 2,5-dichloro-3-fluoropyridine.

**6.** The process as claimed in one or more of claims 1 to 5, wherein the fluoride salts used are potassium fluoride or tetraalkyl($C_1$-$C_{10}$)ammonium fluoride or mixtures with one another or with cesium fluoride, the amount of cesium fluoride in mixtures of this type being not more than 50% by weight.

**7.** The process as claimed in one or more of claims 1 to 6, wherein the fluoride salts used are mixtures of alkali metal fluorides which contain at least 90% by weight of potassium fluoride.

**8.** The process as claimed in claim 7, wherein the mixtures consist of at least 90% by weight of potassium fluoride and at most 10% by weight of cesium fluoride.

**9.** The process as claimed in one or more of claims 1 to 8, wherein the fluoride salt is employed in an amount corresponding to 0.9 to 5 equivalents, preferably 1 or 2 equivalents, per chlorine atom to be exchanged.

**10.** The process as claimed in one or more of claims 1 to 9, wherein the phase transfer catalysts used are quaternary ammonium or phosphonium compounds from the group comprising tetraalkyl-($C_1$-$C_{18}$)ammonium chlorides, bromides or fluorides, tetraalkyl-($C_1$-$C_{18}$)phosphonium chlorides or bromides, tetraphenylphosphonium chloride or bromide, and $(phenyl)_m(alkyl(C_1\text{-}C_{18}))_n$phosphonium chlorides or bromides, where m = 1 to 3, n = 3 to 1 and m + n = 4 .

**11.** The process as claimed in claim 10, wherein the phase transfer catalysts are employed in amounts up to 20% by weight, preferably between 3 and 15% by weight, relative to the amount of alkali metal fluoride.

**12.** The process as claimed in one or more of claims 1 to 9, wherein the phase transfer catalysts employed are oligo- or polyethylene glycol dimethyl ethers which contain 4 to 150 glycol units ($-O-CH_2-CH_2-$) or mixtures thereof with the phase transfer catalysts as claimed in claim 10.

**13.** The process as claimed in claim 12, wherein the oligo- or polyethylene glycol dimethyl ethers are used in amounts from 0.5 to 200% by weight, preferably 5 to 100% by weight, relative to the amount of fluoride salt.

**14.** The process as claimed in claim 2, wherein the polar aprotic solvent used is sulfolane (tetramethylene sulfone), tetramethylene sulfoxide (TMSO), N,N-diethylacetamide, N,N-dimethylacetamide (DMAc), N,N-dimethylformamide (DMF), N-methylpyrrolidone (NMP), dimethyl sulfoxide (DMSO), dimethyl sulfone, diphenyl sulfoxide, diphenyl sulfone, tetramethylurea, tetra-n-butylurea, 1,3-dimethylimidazolidin-2-one (DMI) or mixtures thereof.

15. The process as claimed in one or more of claims 1 to 14, wherein the reaction is carried out at temperatures between 170°C and 230°C.

16. The process as claimed in one or more of claims 1 to 15, wherein the reaction is carried out in the presence of acid-binding agents, preferably alkali metal carbonates or alkaline earth metal carbonates or basic oxides.

17. The process as claimed in claim 16, wherein the use of the acid-binding agents takes place in the fractionation.

18. The process as claimed in claim 16 or 17, wherein the acid-binding agent employed is sodium carbonate and/or potassium carbonate and/or magnesium oxide.

19. The process as claimed in one or more of claims 1 to 18, which is carried out under excess pressure.

20. The process as claimed in one or more of claims 1 to 19, wherein the substituted 2,3-dihalopyridines employed are substituted 2,3-dichloropyridines.

21. The process as claimed in one or more of claims 1 to 20, wherein, in a two-step process, a substituted 2,3-dichloropyridine is first reacted to give the substituted 2-fluoro-3-chloropyridine and the crude mixture obtained in this reaction is reacted again with fluoride salt to give the desired 2,3-difluoropyridine.

**Revendications**

1. Procédé pour la préparation de 2,3-difluoropyridines substituées à l'état fondu ou en présence d'un solvant polaire aprotique ou d'un solvant non polaire, à l'exception du benzonitrile, qui est caractérisé en ce qu'on met à réagir des 2,3-dihalogénopyridines substituées choisies parmi les 2,3-dichloropyridines substituées, les 2-chloro-3-fluoropyridines substituées ou les 3-chloro-2-fluoropyridines substituées avec le fluorure de potassium, de rubidium ou de tétra-alkyle en $C_1$-$C_{18}$-ammonium ou des mélanges de ceux-ci, en mélange avec le fluorure de césium, dans lequel la teneur en fluorure de césium ne se monte pas à plus de 50% en poids, en présence de catalyseurs par transfert de phase à des températures entre 120°C et 250°C, sans éliminer en continu du mélange réactionnel les 2,3-difluoropyridines substituées obtenues.

2. Procédé selon la revendication 1, caractérisé en ce que la réaction se produit dans des solvants polaires aprotiques.

3. Procédé selon la revendication 1 ou 2, caractérisé en ce que les 2,3-halogénopyridines substituées présentent la formule générale (I)

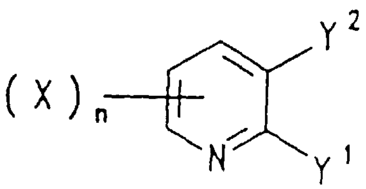

(I)

dans laquelle

X — représente un atome d'halogène, de préférence de chlore ou de brome, ou un autre groupe électro-négatif, de préférence le groupe trifluorométhyle;

$Y^1/Y^2$ — représentent indépendamment l'un de l'autre un atome de fluor ou de chlore, les deux groupes ne représentant pas cependant en même temps un atome de fluor et

n = — 1 ou 2, de préférence 1.

4. Procédé selon la revendication 3, caractérisé en ce que le groupe X (pour n = 1) est situé en position 5 ou 6.

5. Procédé selon la revendication 3 ou 4, caractérisé en ce qu'on emploie comme composés (I) la 2,3,5-trichloropyridine, la 2,3,6-trichloropyridine,
la 2,3,5,6-tétrachloropyridine, la 2,3-dichloro-5-trifluorométhylpyridine, la 2,3-dichloro-5-cyanopyridine, la 3,5-dichloro-2-fluoropyridine ou la 2,5-dichloro-3-fluoropyridine.

**6.** Procédé selon une ou plusieurs des revendications 1 à 5, caractérisé en ce qu'on utilise comme fluorures le fluorure de potassium ou de tétra-alkyle en $C_1$-$C_{18}$-ammonium ou des mélanges de ceux-ci entre eux ou avec le fluorure de césium, dans lequel la teneur en fluorure de césium ne se monte pas à plus de 50% en poids dans de tels mélanges.

**7.** Procédé selon une ou plusieurs des revendications 1 à 6, caractérisé en ce qu'on utilise comme fluorures des mélanges de fluorures de métaux alcalins, qui contiennent au moins 90% en poids de fluorure de potassium.

**8.** Procédé selon la revendication 7, caractérisé en ce que les mélanges sont constitués d'au moins 90% en poids de fluorure de potassium et de 10% en poids au maximum de fluorure de césium.

**9.** Procédé selon la une ou plusieurs des revendications 1 à 8, caractérisé en ce qu'on utilise le fluorure en une quantité correspondant à 0,9 à 5 équivalents, de préférence à 1 à 2 équivalents, par atome de chlore à échanger.

**10.** Procédé selon une ou plusieurs des revendications 1 à 9, caractérisé en ce qu'on utilise comme catalyseurs par transfert de phase des composés d'ammonium ou de phosphonium quaternaires choisis parmi les chlorures, bromures ou fluorures de tétra-alkyle en $C_1$-$C_{18}$-ammonium, les chlorures ou les bromures de tétra-alkyle en $C_1$-$C_{18}$-phosphonium, le chlorure ou le bromure de tétraphényl-phosphonium, les chlorures ou les bromures de (phényl)$_m$(alkyle en $C_1$-$C_{18}$)$_n$-phosphonium, dans lesquels m = 1 à 3, n = 3 à 1 et m +n =4 .

**11.** Procédé selon la revendication 10, caractérisé en ce qu'on utilise les catalyseurs par transfert de phase en quantités jusqu'à 20% en poids, de préférence entre 3 et 15% en poids, par rapport à la quantité de fluorure de

**12.** Procédé selon une ou plusieurs des revendications 1 à 9, caractérisé en ce qu'on utilise comme catalyseurs par transfert de phase des éthers diméthyliques d'oligoou de polyéthylèneglycol, qui contiennent 4 à 150 motifs glycol (-O-$CH_2$-$CH_2$-) ou des mélanges de ceux-ci avec les catalyseurs par transtert de phase selon la revendication 10.

**13.** Procédé selon la revendication 12, caractérisé en ce qu'on utilise les éthers diméthyliques d'oligo- ou de polyéthylèneglycol en quantités de 0,5 à 200% en poids, de préférence de 5 à 100% en poids, par rapport à la quantité de fluorure.

**14.** Procédé selon la revendication 2, caractérisé en ce qu'on utilise comme solvant polaire aprotique le sulfolane (tétraméthylsulfone), le tétra-méthylènesulfoxyde (TMSO), le N,N-diéthylacétamide, le N,N-diméthylacétamide (DMAc), le N,N-diméthylformamide (DMF), la N-méthylpyrrolidone (NMP), le diméthylsulfoxyde (DMSO), la diméthylsulfone, le diphénylsulfoxyde, la diphénylsulfone, la tétraméthylurée, la tétra-n-butylurée, la 1,3-diméthylimidazolidin-2-one (DMI) ou des mélanges de tels solvants.

**15.** Procédé selon une ou plusieurs des revendications 1 à 14, caractérisé en ce qu'on réalise la réaction à des températures entre 170°C et 230°C.

**16.** Procédé selon une ou plusieurs des revendications 1 à 15, caractérisé en ce qu'on travaille en présence d'agents fixateurs d'acide, de préférence des carbonates de métaux alcalins ou alcalino-terreux ou des oxydes à comportement basique.

**17.** Procédé selon la revendication 16, caractérisé en ce que l'emploi des agents fixateurs d'acide est mis en oeuvre au cours du fractionnement.

**18.** Procédé selon la revendication 16 ou 17, caractérisé en ce qu'on utilise comme agent fixateur d'acide le carbonate de sodium et/ou de potassium et/ou l'oxyde de magnésium.

**19.** Procédé selon une ou plusieurs des revendications 1 à 18, caractérisé en ce qu'on travaille en surpression.

**20.** Procédé selon une ou plusieurs des revendications 1 à 19, caractérisé en ce qu'on utilise comme 2,3-dihalogénopyridines substituées des 2,3-dichloropyridines substituées.

**21.** Procédé selon une ou plusieurs des revendications 1 à 20, caractérisé en ce qu'on transforme d'abord, dans un procédé en deux étapes, une 2,3-dichloropyridine substituée d'abord une 2-fluoro-3-chloropyridine substituée et le mélange brut alors obtenu de nouveau avec un fluorure pour obtenir la 2,3-difluoropyridine souhaitée.